# EUROPÄISCHE PATENTSCHRIFT

(11) **EP 1 762 221 B1**
(45) Veröffentlichungstag und Bekanntmachung des Hinweises auf die Patenterteilung: **11.09.2013**
(21) Anmeldenummer: 06117447.0
(22) Anmeldetag: 19.07.2006
(51) Int. Cl.: A61K 8/49, A61Q 17/02, A61K 8/02, A01N 47/16, A01N 25/24

(54) **Insektenabwehrmittel mit Langzeitwirkung**
Insect repellent with long lasting affect
Insectifuge à longue durée

(30) Priorität: 20.07.2005 DE 102005033844
(43) Veröffentlichungstag der Anmeldung: 14.03.2007
(73) Patentinhaber: Beiersdorf AG, 20253 Hamburg (DE)
(72) Erfinder: Kröpke, Rainer, 22869, Schenefeld (DE); Nielsen, Jens, 25448, Henstedt-Ulzburg (DE); Schulz, Jens, 22869, Schenefeld (DE); Schäfer, Andreas, 22299, Hamburg (DE)

(56) Entgegenhaltungen:
- WO-A-02/43489
- WO-A-2004/060063
- WO-A1-02/43490
- WO-A1-02/43491
- WO-A2-02/43656
- WO-A2-03/020232

## Beschreibung

Die vorliegende Erfindung betrifft eine kosmetische Zubereitung enthaltend 1-Piperidincarboxylsäure 2-(2-hydroxyethyl)-1-methylpropylester (INN: Icaridin) und einen oder mehrere Filmbildner sowie die Verwendung von Filmbildnern in Insektenabwehrmitteln.

Insektenschutzmittel (Insektenvertreibungsmittel, Insektenabwehrmittel, Repellentien, Repellents) sind Präparate, die zur Abwehr und/oder Vertreibung von Insekten, aber auch Zecken und Milben äußerlich angewendet werden und verhindern sollen, dass diese auf der Haut aktiv werden. Insektenabwehrmittel sollen die Haut vor Belästigung durch Blut saugende oder beißende Insekten und andere Parasiten und/oder Lästlinge schützen, indem sie diese abwehren, bevor sie sich auf der Haut niederlassen. Die Mittel wirken dementsprechend nicht als Kontaktgifte, sondern nur als Abwehrmittel, da sie die Tiere nicht töten, sondern lediglich vertreiben.

Demgemäß sind im Sinne der vorliegenden Erfindung unter dem Begriff "Insektenschutzmittel" nicht nur solche Präparate zu verstehen, die gegen Insekten (insbesondere Mücken) wirksam sind. Vielmehr gilt das nachstehend Gesagte selbstverständlich auch für solche Präparate, die andere Blut saugende oder beißende Parasiten und/oder Lästlinge (z.B. Spinnen, Flöhe, Milben) abwehren oder vertreiben, auch wenn dies im Einzelfall nicht erwähnt sein mag.

Schon seit Urzeiten werden die Menschen von stechenden oder beißenden Insekten oder anderen Parasiten geplagt. Dementsprechend alt ist das Bedürfnis der Menschheit nach Insektenabwehrmitteln. Eine schon seit der Frühgeschichte bekannte Methode, lästigen oder schädlichen Insekten ihren Aufenthalt in der Nähe des Menschen unattraktiv oder unangenehm zu machen, ist das Anzünden von Feuern mit aromatisch oder streng riechenden Kräutern oder Hölzern und starker Rauchentwicklung. Auch die Behandlung der Haut mit stark riechenden Substanzen zur Abwehr von Insekten ist bereits seit der Antike bekannt. Um die letzte Jahrhundertwende war eine Reihe natürlicher etherischer Öle als 02241 Wz

Insektenabwehrmittel im Gebrauch, so beispielsweise Anisöl, Bergamottöl, Birkenholzteer, Campher, Citronellöl, Eucalyptusöl, Geraniumöl, Kiefernöle, Kokosnußöl, Lavendelöl, Muskatnußöl, Nelkenöl, Orangenblütenöl, Pfefferminzöl, Poleiöle (Pennyroyalöl), Pyrethrum, Thymianöl und Zimtöl.

Wegen ihrer trotz intensiven Geruchs unzureichenden Wirksamkeit und ihrer zum Teil mangelnden Verträglichkeit in höheren Konzentrationen wurden diese Stoffe in heutigen Insektenabwehrmitteln weitgehend durch besser wirksame synthetische Substanzen verdrängt. Es handelt sich dabei überwiegend um hoch siedende Flüssigkeiten oder niedrig schmelzende bzw. sublimierende kristalline Stoffe, die bei Raumtemperatur langsam verdampfen. Die meisten Repellent-Wirkstoffe gehören den Stoffklassen der Amide, Alkohole, Ester und Ether an.

Ein moderner Repellent-Wirkstoff ist beispielsweise der 1-Piperidincarboxylsäure 2-(2-hydroxyethyl)-1-methylpropylester (INN: Icaridin, CAS-Nummer: 119515-38-7, Elincs-Nummer: 423-210-8). Dieser weist die folgende Struktur auf:

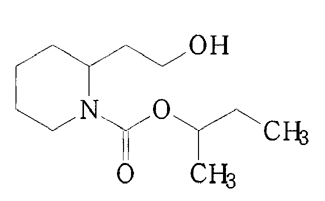

Insektenabwehrmittel des Standes der Technik, die mit 1-Piperidincarboxylsäure 2-(2-hydroxyethyl)-1-methylpropylester formuliert werden, weisen jedoch eine Reihe von Nachteilen auf:
- Die Zubereitungen sind nur eine begrenzte Zeit wirksam (meist 2-6 Stunden). Danach ist der größte Teil des Wirkstoffes verflogen. Das Wirkstoff-Freisetzungsprofil (Freisetzungskinetik) ist dabei relativ ungünstig, da nach einer Anfangsperiode, in der nur unwirksame Mengen des Wirkstoffes freigesetzt werden, über eine längere Periode eine Überschussmenge (d.h. mehr als für die Abschreckung der Insekten eigentlich erforderlich) an Icaridin an die Umwelt abgegeben wird. Icaridin ist ein relativ aufwendig herzustellender (und damit teurer) Wirkstoff, der in hohen Konzentrationen in Einzelfällen bei besonders sensiblen Menschen zu Hautreizungen führen kann. Daher besteht das dringende Bedürfnis, die eingesetzte Wirkstoffmenge optimal zu nutzen bzw. auf das notwendige Maß zu reduzieren.
- Die Zubereitungen lassen sich aufgrund ihrer rheologischen Eigenschaften relativ schwer ihren Verpackungsbehältnissen entnehmen und auf der Haut gleichmäßig verteilen.

Es war daher die Aufgabe der vorliegenden Erfindung, die Mängel des Standes der Technik zu beseitigen. Insbesondere war es die Aufgabe der vorliegenden Erfindung, Insektenabwehrmittel zu entwickeln, die, bei gleichem Wirkstoffgehalt an Icaridin über einen längeren Zeitraum wirksam sind bzw. mit einer reduzierten Menge an Icaridin bei gleicher Wirksamkeitsdauer wie die Produkte des Standes der Technik wirksam sind. Ferner war es die Aufgabe der vorliegenden Erfindung, die Entnahme des Insektenabwehrmittels aus dem Vorratsgefäß und die Verteilbarkeit derselben auf der Haut zu verbessern.

Überraschend gelöst werden die Aufgaben durch eine kosmetische Zubereitung in Form einer O/W-Emulsion enthaltend
a) 1-Piperidincarboxylsäure 2-(2-hydroxyethyl)-1-methylpropylester (INN: Icaridin) in einer Konzentration von 0,1 bis 40 Gewichts-% und
b) einen oder mehrere Filmbildner in einer Gesamtmenge von 0,75 bis 3,5 Gewicht-%, jeweils bezogen auf das Gesamtgewicht der Zubereitung, dadurch gekennzeichnet, dass als

Filmbildner eine oder mehrere Verbindungen gewählt aus der Gruppe der Verbindungen Polyacrylat, Natriumpolyacrylat, Polysaccharide eingesetzt werden.

Ferner werden die Aufgaben überraschend gelöst durch die Verwendung von Filmbildnern gewählt aus der Gruppe der Verbindungen Polycrylat, Natriumpolyacrylat, Polysaccharide zur Verlängerung der Wirksamkeitsdauer kosmetischer O/W-Emulsionen mit einem Gehalt an 1-Piperidincarboxylsäure 2-(2-hydroxyethyl)-1-methylpropylester (INN: Iraridin).

Zwar kennt der Fachmann die WO 02/43491, WO 02/43656, WO 2004/060063. WO 02/43490, WO 02/43489 und die WO 03/020232, doch konnten diese Schriften nicht den Weg zur vorliegenden Erfindung weisen.

Es ist erfindungsgemäß bevorzugt, 1-Piperidincarboxylsäure 2-(2-hydroxyethyl)-1-methylpropylester (INN: Icaridin) in einer Konzentration von 10 bis 20 Gewichts-%, bezogen auf das Gesamtgewicht der Zubereitung, einzusetzen.

Ferner ist es erfindungsgemäß, einen oder mehrere Filmbildner in einer Gesamtmenge von 0,75 bis 3,5 Gewicht-%, bezogen auf das Gesamtgewicht der Zubereitung, einzusetzen.

Es ist vorteilhaft im Sinne der vorliegenden Erfindung, wenn das Gewichtsverhältnis von 1-Piperidincarboxylsäure 2-(2-hydroxyethyl)-1-methylpropylester zur Gesamtmenge an Filmbildnern in der Zubereitung von 10 : 1 bis 1 : 5 beträgt.

Erfindungsgemäß vorteilhafte Ausführungsformen der vorliegenden Erfindung sind dadurch gekennzeichnet, dass als Filmbildner ein polymeres Elektrolyt eingesetzt wird.

Erfindungsgemäß vorteilhafte Ausführungsformen der vorliegenden Erfindung sind dadurch gekennzeichnet, dass die Zubereitung eine Viskosität von 100 bis 16000 mPas aufweist.

Die erfindungsgemäße Viskosität wurde mit einem Haake ViscoTester 02 Viskosimeter gemessen. (Dieser Viskosimetertyp besteht aus einem rotierenden Zylinder und einem ruhenden Messbecher. Über die Rotationswiderstandskraft der Formulierung ermittelt das Gerät die Viskosität der Probe. Drehzahl (rpm)= 62,5. Viskosität nach 30 s. ermitteln. Messtemperatur =25 °C, Drehkörper 2, Skala 1).

Erfindungsgemäß bevorzugte Ausführungsformen der vorliegenden Erfindung liegen insbesondere dann vor, wenn die erfindungsgemäße Zubereitung thixotrop ist.

Die Zubereitung kann erfindungsgemäß vorteilhaft in Form einer dünnflüssigen, sprühfähigen wässrigen oder wässrig-alkoholischen Lösung, in Form eines Gels, als Salbe, Creme oder Lotion (ggf. sprühbar) vorliegen.

Die Zubereitung kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Zubereitung, erfindungsgemäß.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel.

Eine eventuell vorhandene Ölphase kann alle herkömmlichen Bestandteile von in der Kosmetik eingesetzten ÖI-, Fett- und Wachskomponenten enthalten.

Als Emulgatoren können alle aus der Kosmetik bekannten Emulgatoren und Emulgatorsysteme eingesetzt werden.

Neben den erfindungsgemäßen Repellent-Wirkstoffen kann die erfindungsgemäße Zubereitung weitere kosmetische Wirk- und Pflegestoffe enthalten, beispielsweise die nach der Kosmetikverordnung zugelassenen UV-Lichtschutzfilter, und Konservierungsmittel bzw. Konservierungshelfer. Derartige Wirkstoffe können erfindungsgemäß vorteilhaft in Konzentrationen von 0,01 bis 30 Gew-%, bezogen auf das Gesamtgewicht der Zubereitung in dieser enthalten sein.

Als weitere Pflegestoffe können insbesondere Niacinamid, Panthenol, Aloe vera, Hammamelis-Extrakt, Polidocanol, Vitamin E, Vitamin E-Derivate, Vitamin A, Vitamin A-Derivate, Vitamin C, Vitamin C-Derivate, Coenzym Q10, Kreatin, Taurin und/oder Alpha-Glycosylrutin in Konzentrationen von 0,1 bis 30 Gewichts-%, bezogen auf das Gesamtgewicht der Zubereitung eingesetzt werden.

Es ist erfindungsgemäß vorteilhaft, wenn die erfindungsgemäße Zubereitung einen pH-Wert von 3 bis 8 aufweist. Der erfindungsgemäße pH-Wert wird erfindungsgemäß bevorzugt durch den Zusatz von Alpha-Hydroxysäuren und/oder deren Salze (Milchsäure/Lactate oder Zitronensäure/Citrate), Phosphat-Puffer, Kaliumhydroxid, Triethanolamin und/oder Natriumhydroxid eingestellt.

Die erfindungsgemäße Zubereitung wird erfindungsgemäß vorteilhaft in einem Packmittel aufbewahrt. Das erfindungsgemäße Packmittel liegt erfindungsgemäß vorteilhaft in Form einer Tube oder einer Kunststoff-Flasche vor. Dabei sind Kunststoff-Flaschen erfindungsgemäß bevorzugt. Eine erfindungsgemäß besonders bevorzugte Ausführungsform stellen Kunststoff-Flaschen mit aufgesetztem Pumpspender dar, mit welchem die erfindungsgemäße Zubereitung aus dem Packmittel gefördert und ggf. versprüht werden kann.

Es ist erfindungsgemäß bevorzugt, wenn das erfindungsgemäße Kosmetikum aus einem Packmittel aus Polyethylen (PE) gebildet wird. Erfindungsgemäß besonders bevorzugt ist dabei der Einsatz von "high density polyethylene"(HDPE nach DIN 7728, TI. 1, 01/1988).

Erfindungsgemäß ist insbesondere die Verwendung der erfindungsgemäßen Zubereitung zur Prophylaxe von Mückenstichen und Zeckenbissen.

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

**O/W-Emulsion**

| | **1** | **2** | **3** | **4** | **5** |
|---|---|---|---|---|---|
| Polyethylenglycol(21)stearylether | 1 | -- | 2,5 | 2 | 1,5 |
| Polyethylenglycol(2)stearylether | 1 | -- | 5,5 | 3 | 7,5 |
| Cetearylglucosid | --- | 8 | --- | --- | --- |
| Behenylalkohol | 3 | 2 | --- | 1 | --- |
| Stearylalkohol | 3 | 2 | --- | 2 | --- |
| Cetylstearylalkohol | 3 | 4 | --- | --- | 2 |
| hydriertes Polyisobuten | 0,5 | 0,75 | 1,0 | 2,0 | 0,25 |
| Icaridin | 10 | 20 | 15 | 7,5 | 5,0 |
| Octyldodecanol | 0,5 | 1,0 | 0,75 | 3,0 | 0,25 |
| Glycerin | 5 | 10 | 15 | 3 | 7,5 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| Polyacrylat | 1 | 2 | 1,5 | 3 | 0,5 |
| Wasser | ad 100 | ad 100 | ad 100 | ad 100 | ad 100 |

**O/W-Emulsion**

| | **6** | **7** | **8** | **9** | **10** |
|---|---|---|---|---|---|
| Glycerylsteratcitrat | 1,0 | 0,5 | 1,5 | 9,0 | 0,3 |
| Polyethylenglycol(20)cetearylether | 10,0 | 1,0 | 5 | -- | -- |
| Triglycerinmethylglucosedistearat | --- | --- | 3,5 | --- | 2,5 |
| Polysaccharide | 0,75 | 1,5 | 2,25 | 12 | 3,75 |
| Icaridin | 7,5 | 15 | 22,5 | 30 | 37,5 |
| Dimethicon | 0,5 | 3,0 | 0,75 | 1,5 | 0,2 |
| Behenylalkohol | 1 | --- | 2 | --- | 0,2 |
| Dicaprylylcarbonat | 3 | 5 | 10 | 5 | 5 |
| Stearylalkohol | --- | --- | --- | 1 | 0,2 |
| Cetylstearylalkohol | --- | --- | 1 | 1 | 0,2 |
| Tocopherol | 0,5 | 0,5 | 0,75 | 0,25 | 0,1 |
| Octyldodecanol | 0,5 | --- | 0,75 | 3,0 | 0,25 |
| Panthenol | 0,5 | --- | 0,75 | 0,25 | 0,1 |
| Parfum | q,s, | q,s, | q,s, | q,s, | q,s, |
| Caprylic/Capric Triglycerid | 1 | 5 | 3 | 5 | 10 |
| Methylparaben | 0,4 | 0,3 | 0,05 | 0,3 | 0,4 |
| Propylparaben | 0,3 | --- | 0,25 | 0,15 | --- |
| Iodopropynylbutylcarbamat | --- | --- | 0,05 | --- | 0,1 |
| Phenoxyethanol | --- | 0,5 | --- | 0,15 | --- |
| Sorbitol | 10 | --- | -- | 5 | --- |
| Butylenglykol | --- | --- | --- | 5 | 10 |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

**Pumpspray**

| | **11** | **12** | **13** | **14** | **15** |
|---|---|---|---|---|---|
| Polyethylenglycol(40)stearat | 1,0 | --- | 0,5 | -- | -- |
| Cyclomethicon | 0,5 | --- | --- | --- | --- |
| Icaridin | 2 | 10 | 15 | 20 | 40 |
| Parfum | q,s, | q,s, | q,s, | q,s, | q,s, |
| Ethanol | 15 | 20 | 30 | 40 | 20 |
| Methylparaben | 0,4 | 0,1 | 0,05 | 0,3 | 0,4 |
| Meeresextrakt | --- | --- | 0,1 | --- | --- |
| Aloe Vera Extrakt | 0,1 | --- | 0,1 | --- | --- |
| Hammamelis-Extrakt | 0,1 | --- | 0,1 | 5 | 0,5 |
| Glycerin | 5 | 10 | 3 | 15 | 7,5 |
| Natriumpolyacrylat | 1,0 | 2,5 | 3,5 | 0,15 | 7,5 |
| Wasser | ad 100 | ad 100 | ad 100 | ad 100 | ad 100 |

## Patentansprüche

1. Kosmetische Zubereitung in Form einer O/W-Emulsion enthaltend
a) 1-Piperidincarboxylsäure 2-(2-hydroxyethyl)-1-methylpropylester (INN: Icaridin) in einer Konzentration von 0,1 bis 40 Gewichts-% und
b) einen oder mehrere Filmbildner in einer Gesamtmenge von 0,75 bis 3,5 Gewicht-%, jeweils bezogen auf das Gesamtgewicht der Zubereitung, **dadurch gekennzeichnet, dass** als Filmbildner eine oder mehrere Verbindungen gewählt aus der Gruppe der Verbindungen Polyacrylat, Natriumpolyacrylat, Polysaccharide eingesetzt werden.

2. Verwendung von Filmbildnern gewählt aus der Gruppe der Verbindungen Polyacrylat, Natriumpolyacrylat, Polysaccharide zur Verlängerung der Wirksamkeitsdauer kosmetischer O/W-Emulsionen mit einem Gehalt an 1-Piperidincarboxylsäure 2-(2-hydroxyethyl)-1-methylpropylester (INN: Icaridin).

3. Kosmetische Zubereitung nach Anspruch 1 oder Verwendung nach Anspruch 2, **dadurch gekennzeichnet, dass** als Filmbildner ein polymeres Elektrolyt eingesetzt wird.

4. Kosmetische Zubereitung nach einem der vorhergehenden Ansprüche, **dadurch gekennzeichnet, dass** die Zubereitung eine Viskosität von 100 bis 16000 mPas aufweist.

5. Kosmetische Zubereitung nach einem der vorhergehenden Ansprüche, **dadurch gekennzeichnet, dass** die Zubereitung einen pH-Wert von 3 bis 8 aufweist.

6. Kosmetische Zubereitung nach einem der vorhergehenden Ansprüche, **dadurch gekennzeichnet, dass** die Zubereitung thixotrop ist.

7. Packmittel aus Polyethylen, Polypropylen oder Polyethylenterephthalat enthaltend eine kosmetische Zubereitung nach einem der vorhergehenden Ansprüche.

8. Pflaster oder Tuch getränkt mit einer kosmetischen Zubereitung nach einem der vorhergehenden Ansprüche.

## Claims

1. Cosmetic preparation in the form of an O/W emulsion comprising
a) 2-(2-hydroxyethyl)-1-methylpropyl 1-piperidine-carboxylate (INN: Icaridin) in a concentration of from 0.1 to 40% by weight and
b) one or more film formers in a total amount of from 0.75 to 3.5% by weight, in each case based on the total weight of the preparation, **characterized in that** one or more compounds selected from the group of the compounds polyacrylate, sodium polyacrylate, polysaccharides are used as film formers.

2. Use of film formers selected from the group of the compounds polyacrylate, sodium polyacrylate, polysaccharides for extending the efficacy time of cosmetic O/W emulsions with a content of 2-(2-hydroxyethyl)-1-methylpropyl 1-piperidine-carboxylate (INN: Icaridin).

3. Cosmetic preparation according to Claim 1 or use according to Claim 2, **characterized in that** a polymeric electrolyte is used as film former.

4. Cosmetic preparation according to one of the preceding claims, **characterized in that** the preparation has a viscosity of from 100 to 16 000 mPas.

5. Cosmetic preparation according to one of the preceding claims, **characterized in that** the preparation has a pH of from 3 to 8.

6. Cosmetic preparation according to one of the preceding claims, **characterized in that** the preparation is thixotropic.

7. Pack made of polyethylene, polypropylene or polyethylene terephthalate comprising a cosmetic preparation according to one of the preceding claims.

8. Plaster or wipe impregnated with a cosmetic preparation according to one of the preceding claims.

## Revendications

1. Préparation cosmétique sous forme d'une émulsion H/E, contenant
a) de l'ester 2-(2-hydroxyéthyl)-1-méthylpropylique de l'acide 1-pipéridinecarboxylique (INN : Icaridin) en une concentration de 0,1 à 40% en poids et
b) un ou plusieurs agents filmogènes en une quantité totale de 0,75 à 3,5% en poids, à chaque fois par rapport au poids total de la préparation, **caractérisée en ce qu'**on utilise, comme agent filmogène, un ou plusieurs composés choisis dans le groupe des composés polyacrylate, polyacrylate de sodium, polysaccharides.

2. Utilisation d'agents filmogènes choisis dans le groupe des composés polyacrylate, polyacrylate de sodium, polysaccharides pour allonger la durée d'activité d'émulsions cosmétiques H/E présentant une teneur en ester 2-(2-hydroxyéthyl)-1-méthylpropylique de l'acide 1-pipéridinecarboxylique (INN : Icaridin).

3. Préparation cosmétique selon la revendication 1 ou utilisation selon la revendication 2, **caractérisée en ce qu'**on utilise, comme agent filmogène, un électrolyte polymère.

4. Préparation cosmétique selon l'une quelconque des revendications précédentes, **caractérisée en ce que** la préparation présente une viscosité de 100 à 16 000 mPa.s.

5. Préparation cosmétique selon l'une quelconque des revendications précédentes, **caractérisée en ce que** la préparation présente un pH de 3 à 8.

6. Préparation cosmétique selon l'une quelconque des revendications précédentes, **caractérisée en ce que** la préparation est thixotropique.

7. Emballage en polyéthylène, en polypropylène ou en poly(téréphtalate d'éthylène) contenant une préparation cosmétique selon l'une quelconque des revendications précédentes.

8. Pansement ou tissu imbibé d'une préparation cosmétique selon l'une quelconque des revendications précédentes.
